(19) Europäisches Patentamt
European Patent Office
Office européen des brevets

(11) EP 2 191 871 A1

(12) EUROPEAN PATENT APPLICATION
published in accordance with Art. 153(4) EPC

(43) Date of publication:
**02.06.2010 Bulletin 2010/22**

(21) Application number: **08834593.9**

(22) Date of filing: **18.09.2008**

(51) Int Cl.:
***A62D 3/30*** *(2007.01)* ***A62D 3/176*** *(2007.01)*
***A62D 3/37*** *(2007.01)* ***B09B 3/00*** *(2006.01)*
***C02F 1/70*** *(2006.01)* ***C02F 11/00*** *(2006.01)*
***C07F 15/06*** *(2006.01)* ***C07H 23/00*** *(2006.01)*
***A62D 101/43*** *(2007.01)*

(86) International application number:
**PCT/JP2008/002566**

(87) International publication number:
**WO 2009/041002 (02.04.2009 Gazette 2009/14)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **25.09.2007 JP 2007246470**

(71) Applicant: **Nippon Sheet Glass Company Limited**
**Tokyo 108-6321 (JP)**

(72) Inventors:
- **NAKAMURA, Koichiro**
  **Tokyo 108-6321 (JP)**
- **HISHINUMA, Akihiro**
  **Tokyo 108-6321 (JP)**

(74) Representative: **Fiussello, Francesco et al**
**Studio Torta S.r.l.**
**Via Viotti, 9**
**10121 Torino (IT)**

# (54) METHOD FOR DETOXIFYING TOXIC COMPOUND

(57) It is an object of the present invention to provide a beneficial method of detoxifying a harmful compound in order to detoxify the harmful compound containing arsenic etc. effectively.

A method of detoxifying a harmful compound according to the present invention is **characterized in that** a harmful compound containing at least one element selected from the group comprising arsenic, antimony and selenium is detoxified by an exposure to light and / or a heating under the presence of a cobalt complex. In a preferred embodiment of the method of detoxifying a harmful compound according to the present invention, the method is **characterized in that** the harmful compound is detoxified by an alkylation of arsenic, antimony and selenium.



Printed by Jouve, 75001 PARIS (FR)

## Description

### Technical Field

The present invention relates to a method of detoxifying a harmful compound, especially a method of detoxifying a harmful compound wherein the harmful compound is detoxified by an exposure to light.

### Background Art

The heavy metal material such as arsenic, antimony and selenium is widely used as an industrial material, for example, semiconductor, but the influence on the organism by being flowed it out into an environment is concerned, since it is harmful material for the organism.

In the past, as a method for treating these heavy metal, a method wherein a flocculating agent such as polychlorinated aluminum (PAC) is added into the wastewater containing an inorganic arsenic such as a harmful arsenous acid, and then the inorganic arsenic is removed by the filtration after the inorganic arsenic is aggregated, adsorbed to the flocculating agent and iron contained in a raw water and then precipitated, or a method wherein an arsenic compound etc. is adsorbed by using an activated alumina, cerium based flocculating agent, are generally known.

On the other hand, it is known in nature that an inorganic arsenic is stored in sea food such as a seaweed, and then a part of the inorganic arsenic is converted to an organic arsenic compound such as dimethyl arsenic by the physiological response (Nonpatent literature 1 : Kaise et al., 1998, Organomet. Chem., 12 137-143). And it is generally known that these organic arsenic compound has lower toxicity than that of the inorganic arsenic for the mammal.

Nonpatent literature 1 : Kaise et al., 1998, Organomet. Chem., 12 137-143

### Disclosure of the Invention

### Problems to be resolved by the invention

However, in the above method of removing the heavy metal characterized by the use of the filtration and adsorption, it is necessary to store or reclaim a polluted sludge containing the harmful compound such as the inorganic arsenic which is still harmful, and an absorbent to which the harmful compound is absorbed, under the condition of sealing off the harmful compound with the use of the concrete etc., in order to prevent it from being leaked to the outside. Therefore, there is problem that the mass disposal is difficult since a storage place or a large space for a reclaimed area are required.

Moreover, it is internationally recognized that an arsenic contained in the sea food is a harmless arsenobetaine, in the present invention, it is possible to attain the detoxification by chemically converting the highly toxic inorganic arsenic to the harmless arsenobetaine.

Therefore, it is an object of the present invention to provide a beneficial method of detoxifying a harmful compound in order to detoxify the harmful compound containing arsenic etc. effectively and systematically.

### Means of solving the problems

In order to accomplish the above objects, the present inventors made strenuous studies on the optimal conditions regarding the methylating reaction of the harmful compound, by making an attempt to methylate, specially dimethylate, and more preferably trimethylate a harmful compound containing arsenic etc., by chemical reactions with the use of a cobalt complex. As a result, the inventors discovered the present invention.

That is, a method of detoxifying a harmful compound according to the present invention is **characterized in that** a harmful compound containing at least one element selected from the group comprising arsenic, antimony and selenium is detoxified by an exposure to light and / or a heating under the presence of a cobalt complex.

Furthermore, in a preferred embodiment of the method of detoxifying a harmful compound according to the present invention, the method is **characterized in that** the harmful compound is detoxified by an alkylation of arsenic, antimony and selenium.

Furthermore, in a preferred embodiment of the method of detoxifying a harmful compound according to the present invention, the method is **characterized in that** an alkylation reaction is carried out under an exposure to light and / or a heating.

Furthermore, in a preferred embodiment of the method of detoxifying a harmful compound according to the present invention, the method is **characterized in that** the harmful compound is detoxified under the presence of a reducing agent capable of reducing at least one metal selected from the group comprising arsenic, antimony and selenium.

Furthermore, in a preferred embodiment of the method of detoxifying a harmful compound according to the present invention,

present invention, the method is **characterized in that** the reducing agent is a material having SH group.

Furthermore, in a preferred embodiment of the method of detoxifying a harmful compound according to the present invention, the method is **characterized in that** the material having SH group is at least one selected from the group comprising glutathione, reduced glutathione (GSH), cysteine, S-adenosyl cysteine, sulforaphane, homocysteine and thioglycol.

Furthermore, in a preferred embodiment of the method of detoxifying a harmful compound according to the present invention, the method is **characterized in that** the cobalt complex is methyl complex comprising at least one compound selected from methylcobalamin (methylated vitamin B12, official name: Coa-[α-5,6- dimethylbenz-1H- imidazole-1-yl-Coβ- methylcobamide]), vitamin B12 such as cyanocobalamin, cobalt(II) acetyl acetonate, cobalt(III) acetyl acetonate, cobalt carbonyl (dicobalt octacarbonyl), cobalt(II)1,1,1,5,5,5- hexafluoro acetyl acetonate, cobalt (II) meso-tetra phenyl porphin, hexafluoro phosphoric acid bis (pentamethyl cyclopenta dienyl) cobalt, N, N'-bis (salicylidene) ethylene diamine cobalt(II), bis (2,2,6,6-tetramethyl-3,5-heptanedionato) cobalt(II), (chlorophthalocyaninnato) cobalt(II), chlorotris (triphenylphosphine) cobalt(I), methyl complex of cobalt(II) acetate, cobalt(II) benzoate, cobalt(II) cyanide, cyclohexane cobalt(II) butyrate, 2-cobalt(II) ethylhexanoate, meso-tetramethoxyphenyl porphyrin cobalt(II), cobalt naphthenate, cobalt(II) phthalocyanine, methyl cobalt(III) protoporphyrin IX, cobalt stearate, cobalt(II) sulfamate, (1R, 2R)-(-)-1,2-cyclohexanediamino-N,N'-bis (3,5-di-t-butylsalicylidene) cobalt(II), (1S, 2S)-(+)-1,2-cyclohexanediamino-N,N'-bis (3, 5-di-t-butylsalicylidene) cobalt(II), cyclopentadienyl bis (triphenylphosphine) cobalt(I), cyclopentadienyl cobalt dicarbonyl, dibromo bis (triphenylphosphine) cobalt(II), (tetraaminochloro phthalocyaninnato) cobalt(II), (tetra-t-butyl phthalocyaninnato) cobalt(II), or at least one selected from the group comprising cobalt-methyl complex formed by allowing the cobalt compound to coexist with the alkyl halide, especially methyl halide.

Furthermore, in a preferred embodiment of the method of detoxifying a harmful compound according to the present invention, the method is

**characterized in that** the alkylation is a methylation.

Furthermore, in a preferred embodiment of the method of detoxifying a harmful compound according to the present invention, the method is **characterized in that** the harmful compound is converted to a dimethyl compound, or trimethyl compound by the methylation.

Furthermore, in a preferred embodiment of the method of detoxifying a harmful compound according to the present invention, the method is **characterized in that** the dimethyl compound is dimethyl arsonyl ethanol (DMAE), dimethyl arsonyl acetate (DMAA), dimethylarsinic acid, or arseno sugar.

Furthermore, in a preferred embodiment of the method of detoxifying a harmful compound according to the present invention, the method is **characterized in that** the trimethyl compound is arsenocholine, arsenobetaine, trimethyl arseno sugar or trimethyl arsine oxide.

Effect of Invention

The method of detoxifying a harmful compound according to the present invention has an advantageous effect that a large space such as storage place is not required since it is possible to detoxify the harmful compound without limit. Furthermore, according to the method of the present invention, it has an advantageous effect that the unnecessary byproduct does not generate since it does not use a biological material in itself in a viable condition. Furthermore, according to the present invention, it has an advantageous effect that it is possible to decrease the harmful inorganic arsenic even more with a simple method.

Best Mode for Currying Out the Invention

The method of detoxifying a harmful compound according to the present invention uses a cobalt complex. The cobalt complex used herein is not particularly limited, but an organometallic complex having a cobalt - carbon bond etc., may be recited as an example.

As an example of the organometallic complex having a cobalt - carbon bond may be mentioned below. That is, methylcobalamin (methylated vitamin B12, official name: Coa-[α-5,6- dimethylbenz-1H- imidazole-1-yl-CoB- methylcobamide]) is preferably used. Furthermore, mention may be made of at least one selected from the group comprising the methyl complex of at least one compound selected from vitamin B12 such as cyanocobalamin, cobalt(II) acetyl acetonate, cobalt(III) acetyl acetonate, cobalt carbonyl (dicobalt octacarbonyl), cobalt(II)1,1,1,5,5,5-hexafluoro acetyl acetonate, cobalt(II) meso-tetra phenyl porphin, hexafluoro phosphoric acid bis (pentamethyl cyclopenta dienyl) cobalt, N, N'-bis (salicylidene) ethylene diamine cobalt(II), bis (2,2,6,6-tetramethyl-3,5-heptanedionato) cobalt(II), (chlorophthalocyaninnato) cobalt(II), chlorotris (triphenylphosphine) cobalt(I), methyl complex of cobalt(II) acetate, cobalt(II) benzoate, cobalt(II) cyanide, cyclohexane cobalt(II) butyrate, 2-cobalt(II) ethylhexanoate, meso-tetramethoxyphenyl porphyrin cobalt(II), cobalt naphthenate, cobalt(II) phthalocyanine, methyl cobalt(III) protoporphyrin IX, cobalt stearate, cobalt(II) sulfamate, (1R, 2R)-(-)-1,2-cyclohexanediamino-N,N'-bis (3,5-di-t-butylsalicylidene) cobalt(II), (1S, 2S)-(+)-1,2-cyclohexanediamino-N,N'-bis (3, 5-di-t-butylsalicylidene) cobalt(II), cyclopentadienyl bis (triphenylphosphine) cobalt (I), cyclopentadienyl cobalt dicarbonyl, dibromo bis (triphenylphosphine) cobalt(II), (tetraaminochloro phthalocyaninnato) cobalt(II), (tetra-t-butyl phthalocyaninnato) cobalt(II), or at least one selected from the group comprising cobalt-methyl complex formed by allowing the cobalt compound to coexist with the alkyl halide, especially methyl halide. Methylcobalamin is preferable as the organometallic complex having a cobalt - carbon bond, from the viewpoint that it is possible to make it relatively easy to alkylate the harmful compound containing a harmful inorganic arsenic etc., and covert it to an organic material which has a less toxic.

The reason why the cobalt complex is used, is that the use of the cobalt complex makes it possible to proceed the transmethylation reaction in order to transport the methyl group to arsenic etc. An accomplishment of the methylation of arsenic etc., makes it possible to convert a harmful substance into a more harmless substance. Although it is generally known that a toxicity may be further enhanced by the methylation like an example of mercury or lead etc., in the case of arsenic etc., a toxicity may be considerably reduced by the methylation.

In the present invention, an exposure to light and / or a heating make it possible to proceed the transmethylation reaction. Although a detailed mechanism is unclear, but it is estimated that in the case of the use of methylcobalamin as a cobalt complex, the Co - C bond of the Co - Me group of methylcobalamin which is a factor for the methylation, is cleaved by an exposure to light and / or a heating, and thereby making it easy to translocate the methyl group to an atomic element of arsenic etc.

The conditions of the exposure to light may depend on the common procedure, and are not particularly limited. From a viewpoint that the transmethylation reaction may be enhanced, a light intensity is 0.1 to 1000 mW/$cm^2$, more preferably 1 to 1000mW/$cm^2$. An energy is 1mJ to 100J, preferably 100mJ to 100J. As a wavelength of light which is irradiated, mention may be made of the ultraviolet ray, the visible light ray, the near - infrared ray, the infrared ray, the far - infrared radiation ray. Preferably, an exposure to light of the wavelength, with a central focus on an wavelength of an absorption maximum ($\lambda$max) of the absorption band about a cobalt complex, $\lambda$max$\pm$ 500nm, more preferably, $\lambda$max $\pm$ 250nm, further preferably, $\lambda$max $\pm$ 100nm, makes it possible to effectively proceed the methylating reaction in the present invention.

Further, a condition of a heating is not particularly limited, but a heating temperature is 20 to 250 ˚C, more preferably 50 to 150 ˚C from a viewpoint that the transmethylation reaction may be enhanced.

At this moment, the term "the harmful compound" used herein means a compound which gives any adverse affect to the organism when it is flowed out into the environment and exposed to the organism.

As a harmful compound containing arsenic among the harmful compound, mention may be made of arsenious acid, arsenic pentoxide, arsenic trichloride, arsenic pentachloride, arsenic sulfide compound, cyano arsenic compound, chloro arsenic compound, and other arsenic inorganic salt and or the like. In these arsenic, for example, $LD_{50}$ (50% of the fatal dose in mouse) is less or equal to 20, and therefore, it is generally a poisonous value for the organism.

Further, as a harmful compound containing antimony, mention may be made of antimony trioxide, antimony pentoxide, antimony trichloride, and antimony pentachloride and or the like.

Further, as a harmful compound containing selenium, mention may be made of selenium dioxide and selenium trioxide etc.

In a preferred embodiment, the method of detoxifying a harmful compound according to the present invention may further carried out under the existence of a reducing agent capable of reducing at least one metal selected from the group comprising arsenic, antimony and selenium. The presence of the reducing agent like this makes it possible to further accelerate the alkylation. Although it is thought that a reducing ability for the arsenic or the transmethylation reaction are likely to be a rate controlling in the conversion to the arsenobetaine, it is thought that the conversion to the arsenobetaine etc., may be accelerated by adding those reducing agents. As the reducing agent like this, for example, a material having the SH group may be mentioned, which may be specifically at least one selected from the groups comprising glutathione, reduced glutathione (GSH), cysteine, S - adenosyl cysteine, sulforaphane, homocysteine and thioglycol. Moreover, any combinations of these materials having the SH group may be used. For example, combinations of glutathione + homocysteine, or glutathione + thioglycol etc., may be mentioned.

In the method according to the present invention, a detoxification reaction according to the exposure to light or the heating may be carried out under an adequate buffer solution. Those generally used for the isolation, purification or preservation of the biomedical materials may be used for the buffer solution, and those are not particularly limited, but mention may be made of the buffer solution such as a tris buffer, a phosphate buffer, a carbonic acid buffer, and a boric acid buffer. Furthermore, in a viewpoint that it is possible to attain the detoxification more safely, a pH of the buffer solution is preferably in the range of 5 to 10. A pH of the composition for the alkylation is more preferably less than 9. The composition for the alkylation of the present invention may further contain $H_2O_2$. That is, $H_2O_2$ may be added in a viewpoint that an acute toxicity can be decreased by enhancing the oxidation state (from trivalent to pentavalent).

In a preferred embodiment of the method of detoxifying the harmful compound according to the present invention, in the viewpoint that the 50% of an inhibition of cell growth concentration ($IC_{50}$) or the 50% of a lethal dose ($LD_{50}$) is greater, and thereby being able to attain more detoxification, the detoxification of the harmful compound is preferably

attained by increasing the oxidation number of a valence of the one element contained in the above harmful compound. Specifically, it is possible to increase the oxidation number of a valence of the one element by the alkylation in the method of the present invention as described above. Moreover, it is preferable to convert a trivalent of the oxidation number of a valence to a pentavalent in the case that the element is arsenic or antimony, and it is preferable to convert a tetravalent of the oxidation number of a valence to a hexavalent in the case of selenium.

In the present invention, the detoxification of the harmful compound may be carried out by alkylating the harmful compound. At this moment, the present invention may attain the detoxification by alkylating at least one bond of the one element contained in the above harmful compound. At this moment, as an alkyl group added to the one element, mention may be made of a methyl group, an ethyl group, a propyl group etc. From a viewpoint that it is possible to attain the detoxification more effectively, a methyl group is preferable as an alkyl group.

In the method of detoxifying the harmful compound according to the present invention, from a viewpoint of the safety for the living organism, the 50% of a lethal dose ($LD_{50}$) (an oral toxicity which render a 50% of the fatal dose in mouse) of the compound detoxified by the above alkylation is preferably greater or equal to 1000mg/kg, more preferably greater or equal to 5000mg/kg.

Furthermore, in the method of detoxifying the harmful compound according to the present invention, from a viewpoint of the safety for the living organism, the 50% of an inhibition of cell growth concentration ($IC_{50}$) of the compound detoxified by the above alkylation or arylation is preferably greater or equal to 1000$\mu$M, more preferably greater or equal to 3000$\mu$M. The term "the 50% of an inhibition of cell growth concentration ($IC_{50}$)" used herein means a numerical value which gives a necessary concentration of certain substance in order to block or inhibit a 50% of the 100 cell proliferation with the use of the substance. It shows that the smaller the numerical value of $IC_{50}$, the larger the cytotoxicity. Moreover, $IC_{50}$ was calculated from a result of the examination of the cytotoxicity which gives a plasmid DNA damage under the condition at 37˚C, for 24 hours.

At this moment, $IC_{50}$ of each arsenic compound is shown in the table 1

[Table 1]

| $IC_{50}$ value ($\mu$M) | | | |
|---|---|---|---|
| Arsenic(III) compound | | Arsenic(V) compound | |
| Arsenious acid | 10 | Arsenic acid | 100 |
| MMA(III) | 1 | MA(V) | >6000 |
| DMA(III) | 1 | DMA(V) | 3000 |
| | | TMAO | >6000 |
| Arseno sugar(III) | 500 | Arseno sugar(V) | >6000 |

24h, 37˚C

From the table 1, it is revealed that arseno sugar(III) having a trivalent arsenic(III) has higher cytotoxicity than those of monomethylated arsenic (MMA) and dimethylated arsenic (DMA) having a pentavalent arsenic, but has lower cytotoxicity than those of monomethylated arsenic (MMA), dimethylated arsenic (DMA) having a trivalent, and arsenious acid. On the other hand, it is recognized that monomethylated arsenic(MMA), dimethylated arsenic(DMA) having a trivalent arsenic have higher cytotoxicity than that of arsenious acid (trivalent and pentavalen) , but as a whole, the arsenic(V) compound having a pentavalent arsenic has higher safety for the living organism than that of the arsenic(III) compound having a trivalent arsenic in a viewpoint of the cytotoxicity.

Moreover, $LD_{50}$ of each arsenic compound is shown in the table 2

[Table 2]

| | Chemical species of the arsenic | $LD_{50}$(mg/kg) |
|---|---|---|
| As(III) | Inorganic arsenic(III( valency)) | 4.5 |
| As(V) | inorganic arsenic(V( valency)) | 14-18 |
| MMA | monomethyl arsonic acid | 1,800 |
| DMA | dimethylarsinic acid | 1,200 |

(continued)

| | Chemical species of the arsenic | $LD_{50}$(mg/kg) |
|---|---|---|
| AC | arsenocholine | 6,000 |
| TMAO | trimethylarsineoxide | 10,600 |
| AB | arsenobetaine | 10,000 |

Furthermore, in the method of detoxifying the harmful compound according to the present invention, a biological half-life of the compound detoxified by the above alkylation is preferably less or equal to 8 hours from a viewpoint of the safety for the living organism. In the method of detoxifying the harmful compound according to the present invention, it is preferable to convert the harmful compound to the dimethyl compound or the trimethyl compound by means of the methylation from a viewpoint that they are safer and has a lower toxicity. Moreover, as the dimethyl compound mention may be made of dimethyl arsonyl ethanol (DMAE), dimethyl arsonyl acetate (DMAA), dimethylarsinic acid or arseno sugar. As the trimethyl compound mention may be made of arsenocholine, arsenobetaine, trimethyl arseno sugar or trimethyl arsine oxide.

Example

The present invention will be concretely explained in more detail with reference to Examples, but the invention is not intended to be interpreted as being limited to Examples.

Examples 1 to 8

The Examples 1 to 8 of the present invention will be explained.

40 mg (130 μmol) of the reduced glutathione (GSH) and 10mg (7.4 μmol) of methyl cobalamin (MC), are added into a 50 μL of a buffer solution (40mM Tris-HCl buffer solution, pH8). To this was added 2 μL (2.7 nmol as arsenic trioxide) of arsenic trioxide (which is a trivalent inorganic arsenic) solution (standard solution for an atomic absorption, 100 ppm). The concentrations of each components existing in the reaction solution are as follows: The reduced glutathione (GSH): 2.6 mmol/L, Methyl cobalamin (MC): 0.15 mmol/L, the trivalent inorganic arsenic: 5 nmol/L. The preparation conditions of the reaction reagent are as shown in the table 3. This was added into an incubator for heating and reacted at a predetermined temperature, at a predetermined hours. The reaction conditions are shown in the table 4. After the reaction was terminated, the reaction solution was treated by 10 % of a hydrogen peroxide solution, and diluted 500 fold with an ultrapure water, and carried out qualitative and quantitative analysis by the HPLC-ICP-MS method. The samples are separated into 5 types of chemical species, that is, pentavalent inorganic arsenic, pentavalent monomethyl arsenic (MMA), pentavalent dimethyl arsenic (DMA), pentavalent trimethyl arsenic (TMAO) and tetramethyl arsenic (TeMA), and made an analytical curve by a standard sample and carried out a quantitative analysis. The relative concentrations after the reaction were calculated by the following defined formula.

$$\text{The relative concentration of iAs (V)} = 100\% \times [\text{iAs (V)} / (\text{iAs (V)} + \text{MMA} + \text{DMA} + \text{TMAO} + \text{TeMA})]$$

$$\text{The relative concentration of MMA} = 100\% \times [\text{MMA} / (\text{iAs (V)} + \text{MMA} + \text{DMA} + \text{TMAO} + \text{TeMA})]$$

$$\text{The relative concentration of DMA} = 100\% \times [\text{DMA} / (\text{iAs (V)} + \text{MMA} +$$

DMA + TMAO + TeMA)]

The relative concentration of TMAO = 100% × [TMAO / (iAs (V) + MMA + DMA + TMAO + TeMA)]

The relative concentration of TeMA = 100% × [TeMA / (iAs (V) + MMA + DMA + TMAO + TeMA)]

Moreover, a conversion ratio of arsenic was calculated by the following formula.

A conversion ration = 100% × (The concentration of arsenic after the reaction / The concentration of arsenic before the reaction)

A conversion ration = 100% × [(iAs (V) + MMA + DMA + TMAO + TeMA) / iAs (III)]

The result of this is shown in the table 3. The table 3 shows an additive condition of the reaction reagent.

[Table 3]

| No. | Reaction reagent | | | |
|---|---|---|---|---|
| | Reducing agent | Methylating agent | Solvent | iAs(III) |
| | GSH | MC | Buffer solution | (100ppm) |
| | (mg) | (mg) | (μL) | (μL) |
| Example 1 | 0 | 10 | 50 | 2 |
| Reference ex. 1 | 40 | 0 | 50 | 2 |
| Comparative ex. 1 | 0 | 0 | 50 | 2 |
| Example 2 | 0 | 10 | 50 | 2 |
| Reference ex. 2 | 0 | 10 | 50 | 2 |
| Example 3 | 0 | 10 | 50 | 2 |
| Reference ex. 3 | 0 | 10 | 50 | 2 |
| Example 4 | 0 | 10 | 50 | 2 |
| Reference ex 4 | 0 | 10 | 50 | 2 |
| Example 5 | 40 | 10 | 50 | 10 |
| Reference ex. 5 | 40 | 10 | 50 | 10 |
| Example 6 | 40 | 10 | 50 | 2 |
| Reference ex. 6 | 40 | 10 | 50 | 2 |
| Example 7 | 40 | 10 | 50 | 2 |
| Reference ex. 7 | 40 | 10 | 50 | 2 |

(continued)

| No. | Reaction reagent | | | |
|---|---|---|---|---|
| | Reducing agent | Methylating agent | Solvent | iAs(III) |
| | GSH | MC | Buffer solution | (100ppm) |
| | (mg) | (mg) | (μL) | (μL) |
| Example 8 | 40 | 10 | 50 | 2 |
| Reference ex 8 | 40 | 10 | 50 | 2 |

The reaction conditions are shown in the table 4.

[Table 4]

| No. | Reaction condition | | | |
|---|---|---|---|---|
| | Heating condition | | Exposure condition | |
| | Tem. | Time | Light intensity | Energy |
| | (˚C) | (hr) | (mW/cm2) | (J) |
| Example 1 | 20 | 1 | 5 | 18 |
| Reference ex. 1 | 20 | 1 | 5 | 18 |
| Cornparative ex. 1 | 20 | 1 | 5 | 18 |
| Example 2 | 30 | 2 | 5 | 36 |
| Reference ex. 2 | 30 | 2 | 0 | 0 |
| Example 3 | 50 | 2 | 5 | 36 |
| Reference ex. 3 | 50 | 2 | 0 | 0 |
| Example 4 | 80 | 2 | 5 | 36 |
| Reference ex. 4 | 80 | 2 | 0 | 0 |
| Example 5 | 20 | 2 | 5 | 36 |
| Reference ex. 5 | 20 | 2 | 0 | 0 |
| Example 6 | 30 | 2 | 5 | 36 |
| Reference ex. 6 | 30 | 2 | 0 | 0 |
| Example 7 | 50 | 2 | 5 | 36 |
| Reference ex. 7 | 50 | 2 | 0 | 0 |
| Example 8 | 80 | 2 | 5 | 36 |
| Reference ex. 8 | 80 | 2 | 0 | 0 |

The relative ratio of the reaction product and the conversion ratio are shown in the table 5.

[Table 5]

| No. | Before reaction | After reaction | | | | | | Conv. ratio |
|---|---|---|---|---|---|---|---|---|
| | Raw material | Product(relative yield) | | | | | | Main component / Raw material |
| | iAs(III) | iAs(V) | MMA | DMA | TMA | TeMA | Total | |
| | (ppm) | (%) | (%) | (%) | (%) | (%) | (%) | (%) |
| Example 1 | 4 | 92 | 8 | 0 | 0 | 0 | 100 | 95 |
| Reference ex. 1 | 4 | 100 | 0 | 0 | 0 | 0 | 100 | 93 |

(continued)

| No. | Before reaction | After reaction | | | | | | Conv. ratio |
|---|---|---|---|---|---|---|---|---|
| | Raw material | Product(relative yield) | | | | | | Main component / Raw material |
| | iAs(III) | iAs(V) | MMA | DMA | TMA | TeMA | Total | |
| | (ppm) | (%) | (%) | (%) | (%) | (%) | (%) | (%) |
| Comparative ex. 1 | 4 | 100 | 0 | 0 | 0 | 0 | 100 | ~100 |
| Example 2 | 4 | 87 | 13 | 0 | 0 | 0 | 100 | ~100 |
| Reference ex 2 | 4 | 100 | 0 | 0 | 0 | 0 | 100 | 93 |
| Example 3 | 4 | 91 | 9 | 0 | 0 | 0 | 100 | ~100 |
| Reference ex. 3 | 4 | 96 | 4 | 0 | 0 | 0 | 100 | 99 |
| Example 4 | 4 | 95 | 5 | 0 | 0 | 0 | 100 | ~100 |
| Reference ex. 4 | 4 | 97 | 3 | 0 | 0 | 0 | 100 | ~100 |
| Examples 5 | 20 | 50 | 17 | 21 | 12 | 0 | 100 | 94 |
| Reference ex. 5 | 20 | 51 | 33 | 14 | 2 | 0 | 100 | 96 |
| Example 6 | 4 | 51 | 17 | 18 | 14 | 0 | 100 | 93 |
| Reference ex. 6 | 4 | 56 | 27 | 13 | 4 | 0 | 100 | 92 |
| Example 7 | 4 | 25 | 12 | 15 | 47 | 0 | 100 | 91 |
| Reference ex. 7 | 4 | 36 | 26 | 18 | 20 | 0 | 100 | 94 |
| Example 8 | 4 | 0 | 4 | 3 | 91 | 2 | 100 | 99 |
| Reference ex. 8 | 4 | 0 | 12 | 11 | 76 | 1 | 100 | 99 |

As it is clear from the comparison of the example 1, the reference example 1 and comparative example 1, in the case of no heating under the existence of no reducing agent, it was revealed that arsenic trioxide was converted into monomethylated arsenic (MMA) having a low toxicity by the exposure to light.

Moreover, as it is clear from the comparison of the example 2, the example 3 and the example 4 with the reference example 2, the reference example 3 and the reference example 4 respectively, it was revealed that arsenic trioxide was converted into monomethylated arsenic (MMA) having a low toxicity by the exposure to light in the existence of no reducing agent.

As it is clear from the comparison of the example 5, the example 6, the example 7 and the example 8 with the reference example 5, the reference example 6, the reference example 7 and the reference example 8 respectively, it was revealed that a methylating reaction is accelerated capable of converting arsenic trioxide into monomethylated arsenic (MMA), dimethylated arsenic (DMA), trimethylated arsenic (TMAO) having a low toxicity by the exposure to light in the existence of the reducing agent. Furthermore, as it is clear from the comparison of the example 5, the example 6, the example 7 and the example 8, it was revealed that a methylating reaction is accelerated capable of converting arsenic trioxide into monomethylated arsenic (MMA), dimethylated arsenic (DMA), trimethylated arsenic (TMAO) having a low toxicity under the conditions of the treatment of heating, and the exposure to light in the existence of the reducing agent.

Example 9

Furthermore, an effect of the exposure to light etc., was examined. The table 6 shows the effects of the reducing agent, the exposure to light and temperature for the methylating reaction in the case of the use of iAs (III) as a stating material.

[Table 6]

| Test No | Starting material (As) | Reducing agent | Reaction condition | | | Relative ratio | | | | | | Conv ratio |
|---|---|---|---|---|---|---|---|---|---|---|---|---|
| | | GSH | Temp. | Exposure to light | Time | iAs(V) | MMA | DMA | TMAO | TeMA | Total | |
| | | | (°C) | | (hr) | (%) | (%) | (%) | (%) | (%) | (%) | (%) |
| 1 | iAs(III) | ○ | 30 | ○ | 2 | 51 | 17 | 18 | 14 | 0 | 100 | 93 |
| 2 | iAs(III) | × | 30 | ○ | 2 | 87 | 13 | O | O | 0 | 100 | 103 |
| 3 | iAs(III) | ○ | 30 | × | 2 | 56 | 27 | 13 | 4 | 0 | 100 | 92 |
| 4 | iAs(III) | × | 30 | × | 2 | 100 | 0 | 0 | 0 | 0 | 100 | 93 |
| 5 | iAs(III) | ○ | 50 | ○ | 2 | 25 | 12 | 15 | 47 | 0 | 100 | 91 |
| 6 | iAs(III) | × | 50 | ○ | 2 | 91 | 9 | 0 | 0 | 0 | 100 | 104 |
| 7 | iAs(III) | ○ | 50 | × | 2 | 36 | 26 | 18 | 20 | 0 | 100 | 94 |
| 8 | iAs(III) | × | 50 | × | 2 | 100 | 0 | 0 | 0 | 0 | 100 | 99 |
| 9 | iiAs(III) | ○ | 80 | ○ | 2 | 0 | 4 | 3 | 91 | 2 | 100 | 99 |
| 10 | iAs(III) | × | 80 | ○ | 2 | 95 | 5 | 0 | 0 | 0 | 100 | 101 |
| 11 | iAs(III) | ○ | 80 | × | 2 | 0 | 12 | 11 | 76 | 1 | 100 | 99 |
| 12 | iAs(III) | × | 80 | × | 2 | 100 | 0 | 0 | 0 | 0 | 100 | 103 |
| 13 | iAs(III) | ○ | 100 | ○ | 2 | 0 | 0 | 0 | 93 | 7 | 100 | 100 |
| 14 | iAs(III) | × | 100 | ○ | 2 | 98 | 2 | 0 | 0 | 0 | 100 | 115 |
| 15 | iAs(III) | ○ | 100 | × | 2 | 0 | 0 | 0 | 95 | 5 | 100 | 97 |
| 16 | iAs(III) | × | 100 | × | 2 | 100 | 0 | 0 | 0 | 0 | 100 | 122 |
| 17 | iAs(III) | ○ | 120 | ○ | 2 | 0 | 0 | 0 | 93 | 7 | 100 | 95 |
| 18 | iAs(III) | × | 120 | ○ | 2 | 99 | 1 | 0 | 0 | 0 | 100 | 117 |
| 19 | iAs(III) | ○ | 120 | × | 2 | 0 | 0 | 0 | 93 | 7 | 100 | 93 |
| 20 | iAs(III) | × | 120 | × | 2 | 97 | 2 | 0 | 0 | 1 | 100 | 119 |

In the table 6, iAs(III) : Trivalent arsenic, iAs (V): pentavalent arsenic, MMA : Monomethylated arsenic acid, DMA : Dimethylated arsinic acid, TMAO : Trimethylarsineoxide, TeMA: tetramethylated arsenic acid, GSH : Glutathione (reduced form), concentration of arsenic of the stating arsenic compound: 2.7nmol, GSH (Reduced type of Glutathione): 130 μmol, Methylcobalamin: 7.4 μmol, solvent (tris HCL buffer solution, pH 8) : 50 μL, light energy: 36J (5mW/$cm^2$, 2 hours), respectively. Furthermore, a conversion ratio was calculated by the following formula: A conversion ration (%) = 100% × (The concentration of arsenic after the reaction / The concentration of arsenic before the reaction).

As it is clear from the comparison of the test number 4 and the test number 2, it was revealed that the methylating reaction of the below formula [Chemical 1] is accelerated by the exposure to light, even if there is no reducing agent, GSH.

[Chemical 1]

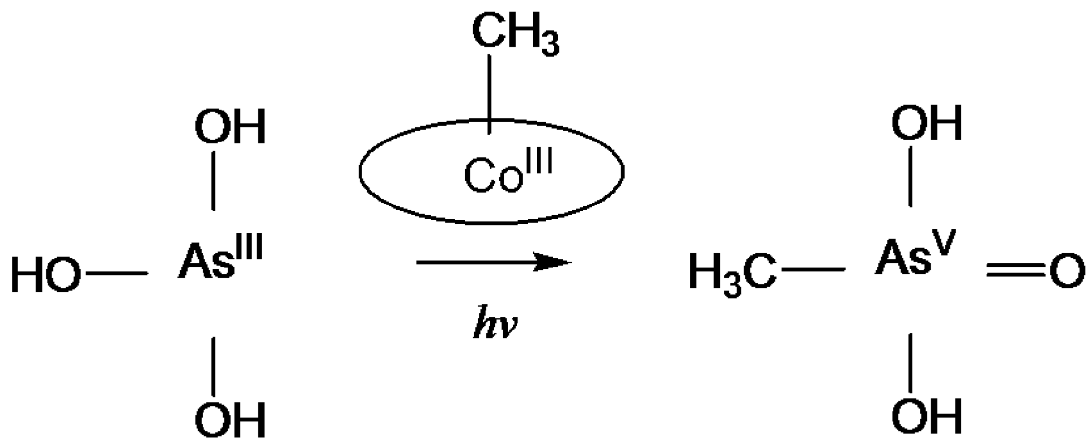


iAs(III) MMA

As it is clear from the comparison of the test number 3 and the test number 1, it was revealed that the methylating reaction of the below formula [Chemical 2] is remarkably accelerated by the exposure to light, in the case of the existence of the reducing agent, GSH.

[Chemical 2]

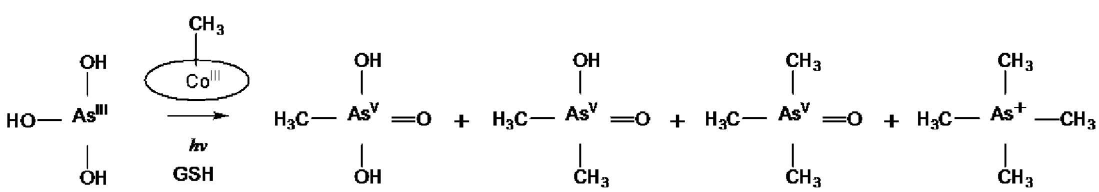


iAs(III) MMA DMA TMAO TeMA

As it is clear from the comparison of the test number 9, the test number 15 and the test number 19, it was revealed that the conditions under the exposure to light at 80˚C make it possible to obtain a harmless TMAO at yield of 90% or more, and it was also revealed that this can obtain almost the same degree of effect as those of the reaction carried out under the conditions of no exposure to light at more higher temperature (100 ˚C, 120˚C) (the test number 15 and the test number 19).

Example 10

Further, the effect related to the exposure to light etc., was also examined about another example. The table 7 shows the effects of the reducing agent, the exposure to light and the temperature for the methylating reaction in the case of the use of iAs (V) as a stating material.

[Table 7]

| Test No | Starting material (As) | Reducing agent | Reaction condition | | | Relative ratio | | | | | | Conv. ratio |
|---|---|---|---|---|---|---|---|---|---|---|---|---|
| | | GSH | Temp. | Exposure to light | Time | iAs(V) | MMA | DMA | TMAO | TeMA | Total | |
| | | | (˚C) | | (hr) | (%) | (%) | (%) | (%) | (%) | (%) | (%) |
| 21 | iAs(V) | ○ | 30 | ○ | 2 | 61 | 19 | 10 | 10 | 0 | 100 | 93 |
| 22 | iAs(V) | × | 30 | ○ | 2 | 100 | 0 | 0 | 0 | 0 | 100 | 104 |
| 23 | iAs(V) | ○ | 30 | × | 2 | 57 | 32 | 9 | 2 | 0 | 100 | 93 |
| 24 | iAs(V) | × | 30 | × | 2 | 100 | 0 | 0 | 0 | 0 | 100 | 105 |
| 25 | iAs(V) | ○ | 50 | ○ | 2 | 34 | 15 | 10 | 42 | 0 | 100 | 93 |
| 26 | iAs(V) | × | 50 | ○ | 2 | 100 | 0 | 0 | 0 | 0 | 100 | 116 |
| 27 | iAs(V) | ○ | 50 | × | 2 | 38 | 35 | 15 | 13 | 0 | 100 | 94 |
| 28 | iAs(V) | × | 50 | × | 2 | 100 | 0 | 0 | 0 | 0 | 100 | 115 |
| 29 | iAs(V) | ○ | 80 | ○ | 2 | 0 | 0 | 1 | 95 | 4 | 100 | 84 |
| 30 | iAs(V) | × | 80 | ○ | 2 | 100 | 0 | 0 | 0 | 0 | 100 | 109 |
| 31 | iAs(V) | ○ | 80 | × | 2 | 0 | 4 | 6 | 86 | 4 | 100 | 88 |
| 32 | iAs(V) | × | 80 | × | 2 | 100 | 0 | 0 | 0 | 0 | 100 | 108 |
| 33 | iAs(V) | ○ | 100 | ○ | 2 | 0 | 0 | 0 | 93 | 7 | 100 | 86 |
| 34 | iAs(V) | × | 100 | ○ | 2 | 100 | 0 | 0 | 0 | 0 | 100 | 108 |
| 35 | iAs(V) | ○ | 100 | × | 2 | 0 | 0 | 0 | 95 | 5 | 100 | 85 |
| 36 | iAs(V) | × | 100 | × | 2 | 100 | 0 | 0 | 0 | 0 | 100 | 110 |
| 37 | iAs(V) | ○ | 120 | ○ | 2 | 0 | 0 | 0 | 93 | 7 | 100 | 87 |
| 38 | iAs(V) | × | 120 | ○ | 2 | 100 | 0 | 0 | 0 | 0 | 100 | 111 |
| 39 | iAs(V) | ○ | 120 | × | 2 | 0 | 0 | 0 | 95 | 5 | 100 | 86 |
| 40 | iAs(V) | × | 120 | × | 2 | 100 | 0 | 0 | 0 | 0 | 100 | 112 |

In the table 7, iAs(III) : Trivalent arsenic, iAs (V): pentavalent arsenic, MMA : Monomethylated arsenic acid, DMA : Dimethylated arsinic acid, TMAO : Trimethylarsineoxide, TeMA: tetramethylated arsenic acid, GSH : Glutathione (reduced form), concentration of arsenic of the stating arsenic compound: 2.7nmol, GSH (Reduced type of Glutathione): 130 umol, Methylcobalamin: 7.4 μmol, solvent (tris HCL buffer solution, pH 8) : 50 μL, light energy: 36J (5mW/cm$^2$, 2 hours), respectively. Furthermore, a conversion ratio was calculated by the following formula: A conversion ration (%) = 100% × (The concentration of arsenic after the reaction / The concentration of arsenic before the reaction).

As it is clear from the comparison of the test number 23 and the test number 21, it was revealed that the methylating reaction of the below formula [Chemical 3] is remarkably accelerated by the exposure to light, in the case of the existence of the reducing agent, GSH.

[Chemical 3]

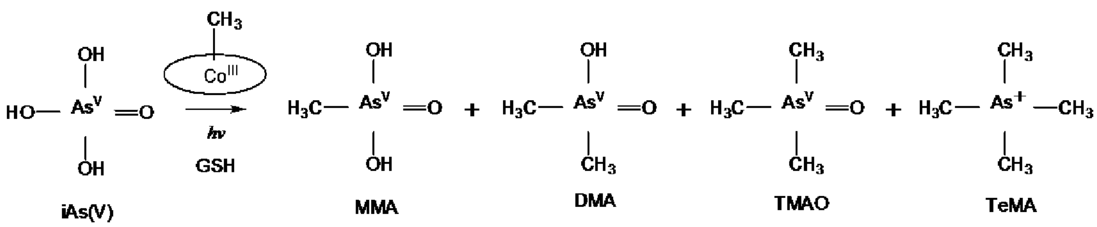


As it is clear from the comparison of the test number 29, the test number 35 and the test number 39, it was revealed that the conditions under the exposure to light at 80˚C make it possible to obtain a harmless TMAO at yield of 90% or more, and it was also revealed that this can obtain almost the same degree of effect as those of the reaction carried out under the conditions of no exposure to light at more higher temperature (100 ˚C, 120˚C) (the test number 35 and the test number 39).

Example 11

Further, the effects related to the exposure to light and the temperature etc., were also examined. The table 8 shows the effects of the reducing agent, the exposure to light and the temperature for the methylating reaction in the case of the use of MMA as a stating material.

[Table 8]

| Test No. | Starting material (As). | Reducing agent | Reaction condition | | | Relative ratio | | | | | | Conv. ratio |
|---|---|---|---|---|---|---|---|---|---|---|---|---|
| | | GSH | Temp, | Exposure to light | Time | iAs(V) | MMA | DMA | TMAO | TeMA | Total | |
| | | | (°C) | | (hr) | (%) | (%) | (%) | (%) | (%) | (%) | (%) |
| 41 | MMA | ○ | 30 | ○ | 2 | 0 | 8 | 30 | 62 | 0 | 100 | 92 |
| 42 | MMA | × | 30 | ○ | 2 | 0 | 100 | 0 | 0 | 0 | 100 | 102 |
| 43 | MMA | ○ | 30 | × | 2 | 0 | 27 | 52 | 20 | 2 | 100 | 104 |
| 44 | MMA | × | 30 | × | 2 | 0 | 100 | 0 | 0 | 0 | 100 | 103 |
| 45 | MMA | ○ | 50 | ○ | 2 | 0 | 3 | 6 | 87 | 4 | 100 | 104 |
| 46 | MMA | × | 50 | ○ | 2 | 0 | 100 | 0 | 0 | 0 | 100 | 108 |
| 47 | MMA | ○ | 50 | × | 2 | 0 | 15 | 31 | 50 | 4 | 100 | 115 |
| 48 | MMA | × | 50 | × | 2 | 0 | 100 | 0 | 0 | 0 | 100 | 109 |
| 49 | MMA | ○ | 80 | ○ | 2 | 0 | 0 | 0 | 89 | 11 | 100 | 113 |
| 50 | MMA | × | 80 | ○ | 2 | 0 | 100 | 0 | 0 | 0 | 100 | 103 |
| 51 | MMA | ○ | 80 | × | 2 | 0 | 1 | 2 | 89 | 8 | 100 | 119 |
| 52 | MMA | × | 80 | × | 2 | 0 | 100 | 0 | 0 | 0 | 100 | 105 |
| 53 | MMA | ○ | 100 | ○ | 2 | 0 | 0 | 0 | 84 | 16 | 100 | 113 |
| 54 | MMA | × | 100 | ○ | 2 | 0 | 100 | 0 | 0 | 0 | 100 | 101 |
| 55 | MMA | ○ | 100 | × | 2 | 0 | 0 | 0 | 86 | 14 | 100 | 115 |
| 56 | MMA | × | 100 | × | 2 | 0 | 100 | 0 | 0 | 0 | 100 | 100 |
| 57 | MMA | ○ | 120 | ○ | 2 | 0 | 0 | 0 | 80 | 20 | 100 | 121 |
| 58 | MMA | × | 120 | ○ | 2 | 0 | 100 | 0 | 0 | 0 | 100 | 109 |
| 59 | MMA | ○ | 120 | × | 2 | 0 | 0 | 0 | 75 | 25 | 100 | 79 |
| 60 | MMA | × | 120 | × | 2 | 0 | 100 | 0 | 0 | 0 | 100 | 111 |

In the table 8, iAs(III) : Trivalent arsenic, iAs (V): pentavalent arsenic, MMA : Monomethylated arsenic acid, DMA : Dimethylated arsinic acid, TMAO : Trimethylarsineoxide, TeMA: tetramethylated arsenic acid, GSH : Glutathione (reduced form), concentration of arsenic of the stating arsenic compound: 2.7nmol, GSH (Reduced type of Glutathione): 130 umol, Methylcobalamin: 7.4 μmol, solvent (tris HCL buffer solution, pH 8) : 50 μL, light energy: 36J (5mW/cm$^2$, 2 hours), respectively. Furthermore, a conversion ratio was calculated by the following formula: A conversion ration (%) = 100% × (The concentration of arsenic after the reaction / The concentration of arsenic before the reaction).

As it is clear from the comparison of the test number 43 and the test number 41, it was revealed that the methylating reaction of the below formula [Chemical 4] is remarkably accelerated by the exposure to light, in the case of the existence of the reducing agent, GSH.

[Chemical 4]

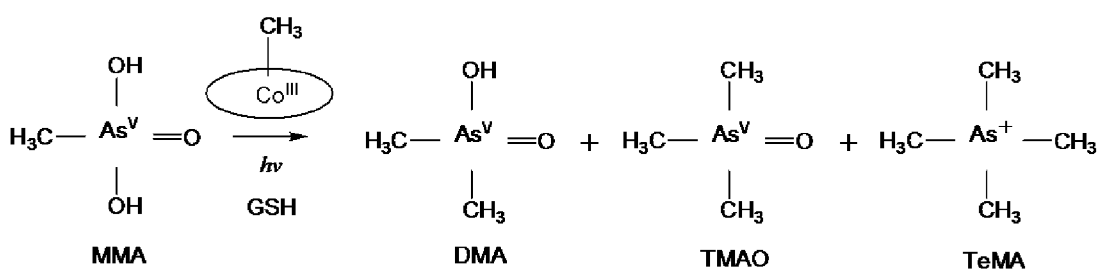


As it is clear from the comparison of the test number 49, the test number 55 and the test number 59, it was revealed that the conditions under the exposure to light at 80˚C make it possible to obtain a harmless TMAO at yield of 80% or more, and it was also revealed that this can obtain almost the same degree of effect as those of the reaction carried out under the conditions of no exposure to light at more higher temperature (100 ˚C, 120˚C) (the test number 55 and the test number 59).

Example 12

Further, the effects related to the exposure to light and the temperature etc., were examined. The table 9 shows the effects of the reducing agent, the exposure to light and the temperature for the methylating reaction in the case of the use of DMA as a stating material.

[Table 9]

| Test No. | Starting material (As) | Reducing agent | Reaction condition | | | Relative ratio | | | | | | Conv. ratio |
|---|---|---|---|---|---|---|---|---|---|---|---|---|
| | | GSH | Temp. | Exposure to light | Time | iAs(V) | MMA | DMA | TMAO | TeMA | Total | |
| | | | (˚C) | | (hr) | (%) | (%) | (%) | (%) | (%) | (%) | (%) |
| 61 | DMA | ○ | 30 | ○ | 2 | 0 | 2 | 29 | 67 | 3 | 100 | 76 |
| 62 | DMA | × | 30 | ○ | 2 | 0 | 0 | 100 | 0 | 0 | 100 | 103 |
| 63 | DMA | ○ | 30 | × | 2 | 0 | 0 | 59 | 38 | 3 | 100 | 89 |
| 64 | DMA | × | 30 | × | 2 | 0 | 0 | 100 | 0 | 0 | 100 | 108 |
| 65 | DMA | ○ | 50 | ○ | 2 | 0 | 0 | 3 | 92 | 5 | 100 | 92 |
| 66 | DMA | × | 50 | ○ | 2 | 0 | 0 | 100 | 0 | 0 | 100 | 103 |
| 67 | DMA | ○ | 50 | × | 2 | 0 | 0 | 28 | 65 | 7 | 100 | 88 |
| 68 | DMA | × | 50 | × | 2 | 0 | 0 | 100 | 0 | 0 | 100 | 97 |
| 69 | DMA | ○ | 80 | ○ | 2 | 0 | 0 | 0 | 83 | 17 | 100 | 106 |
| 70 | DMA | × | 80 | ○ | 2 | 0 | 0 | 100 | 0 | 0 | 100 | 103 |
| 71 | DMA | ○ | 80 | × | 2 | 0 | 0 | 0 | 79 | 21 | 100 | 108 |
| 72 | DMA | × | 80 | × | 2 | 0 | 0 | 100 | 0 | 0 | 100 | 94 |
| 73 | DMA | ○ | 100 | ○ | 2 | 0 | 0 | 0 | 72 | 28 | 100 | 98 |
| 74 | DMA | × | 100 | ○ | 2 | 0 | 0 | 100 | 0 | 0 | 100 | 104 |
| 75 | DMA | ○ | 100 | × | 2 | 0 | 0 | 0 | 70 | 30 | 100 | 108 |
| 76 | DMA | × | 100 | × | 2 | 0 | 0 | 100 | 0 | 0 | 100 | 110 |
| 77 | DMA | ○ | 120 | ○ | 2 | 0 | 0 | 0 | 65 | 35 | 100 | 112 |
| 78 | DMA | × | 120 | ○ | 2 | 0 | 0 | 88 | 2 | 10 | 100 | 120 |
| 79 | DMA | ○ | 120 | × | 2 | 0 | 0 | 0 | 60 | 40 | 100 | 115 |
| 80 | DMA | × | 120 | × | 2 | 0 | 0 | 100 | 0 | 0 | 100 | 112 |

In the table 8, iAs(III) : Trivalent arsenic, iAs (V): pentavalent arsenic, MMA : Monomethylated arsenic acid, DMA : Dimethylated arsinic acid, TMAO : Trimethylarsineoxide, TeMA: tetramethylated arsenic acid, GSH : Glutathione (reduced form), concentration of arsenic of the stating arsenic compound: 2.7nmol, GSH (Reduced type of Glutathione): 130 μmol, Methylcobalamin: 7.4 μmol, solvent (tris HCL buffer solution, pH 8) : 50 μL, light energy: 36J (5mW/$cm^2$, 2 hours), respectively. Furthermore, a conversion ratio was calculated by the following formula: A conversion ration (%) = 100% × (The concentration of arsenic after the reaction / The concentration of arsenic before the reaction).

As it is clear from the comparison of the test number 63 and the test number 61, it was revealed that the methylating reaction of the below formula [Chemical 5] is remarkably accelerated by the exposure to light, in the case of the existence of the reducing agent, GSH.

[Chemical 5]

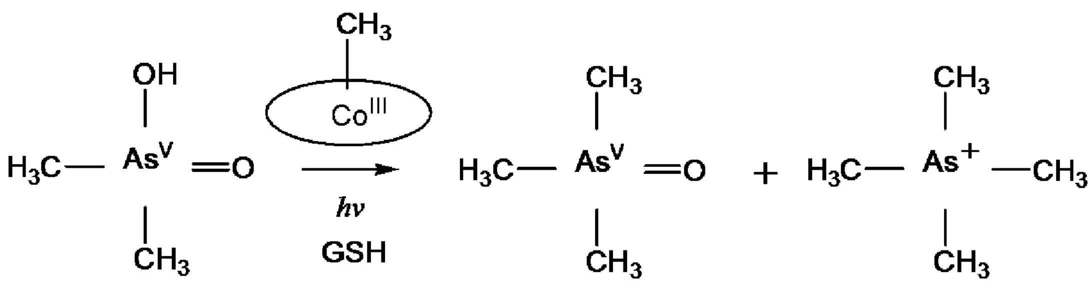


DMA TMAO TeMA

As it is clear from the comparison of the test number 65 and the test number 67, a harmless TMAO at yield of 90% or more can be obtained under the exposure to light at 50˚C.

Example 13

The conditions related to the exposure to light and the temperature etc., were examined. The table 10 shows the effects of the reducing agent, the exposure to light and the temperature for the methylating reaction in the case of the use of TMAO as a stating material.

[Table 10]

| Test No. | Starting material (As) | Reducing agent | Reaction condition | | | Relative ratio | | | | | | Conv. ratio |
|---|---|---|---|---|---|---|---|---|---|---|---|---|
| | | GSH | Temp. | Exposure to light | Time | iAs(V) | MMA | DMA | TMAO | TeMA | Total | |
| | | | (˚C) | | (hr) | (%) | (%) | (%) | (%) | (%) | (%) | (%) |
| 81 | TMAO | ○ | 30 | ○ | 2 | 0 | 0 | 4 | 89 | 7 | 100 | 74 |
| 82 | TMAO | × | 30 | ○ | 2 | 0 | 0 | 0 | 100 | 0 | 100 | 93 |
| 83 | TMAO | ○ | 30 | × | 2 | 0 | 0 | 0 | 90 | 10 | 100 | 73 |
| 84 | TMAO | × | 30 | × | 2 | 0 | 0 | 0 | 100 | 0 | 100 | 95 |
| 85 | TMAO | ○ | 50 | ○ | 2 | 0 | 0 | 0 | 91 | 9 | 100 | 71 |
| 86 | TMAO | × | 50 | ○ | 2 | 0 | 0 | 0 | 100 | 0 | 100 | 83 |
| 87 | TMAO | ○ | 50 | × | 2 | 0 | 0 | 0 | 86 | 14 | 100 | 76 |
| 88 | TMAO | × | 50 | × | 2 | 0 | 0 | 0 | 100 | 0 | 100 | 83 |
| 89 | TMAO | ○ | 80 | ○ | 2 | 0 | 0 | 0 | 82 | 18 | 100 | 80 |
| 90 | TMAO | × | 80 | ○ | 2 | 0 | 0 | 0 | 100 | 0 | 100 | 82 |
| 91 | TMAO | ○ | 80 | × | 2 | 0 | 0 | 0 | 73 | 27 | 100 | 83 |
| 92 | TMAO | × | 80 | × | 2 | 0 | 0 | 0 | 100 | 0 | 100 | 84 |
| 93 | TMAO | ○ | 100 | ○ | 2 | 0 | 0 | 0 | 71 | 29 | 100 | 79 |
| 94 | TMAO | × | 100 | ○ | 2 | 0 | 0 | 0 | 100 | 0 | 100 | 93 |
| 95 | TMAO | ○ | 100 | × | 2 | 0 | 0 | 0 | 62 | 38 | 100 | 89 |
| 96 | TMAO | × | 100 | × | 2 | 0 | 0 | 0 | 100 | 0 | 100 | 92 |
| 97 | TMAO | ○ | 120 | ○ | 2 | 0 | 0 | 0 | 56 | 44 | 100 | 79 |
| 98 | TMAO | × | 120 | ○ | 2 | 0 | 0 | 0 | 100 | 0 | 100 | 94 |
| 99 | TMAO | ○ | 120 | × | 2 | 0 | 0 | 0 | 46 | 54 | 100 | 83 |
| 100 | TMAO | × | 120 | × | 2 | 0 | 0 | 0 | 100 | 0 | 100 | 84 |

In the table 10, iAs(III) : Trivalent arsenic, iAs (V): pentavalent arsenic, MMA : Monomethylated arsenic acid, DMA : Dimethylated arsinic acid, TMAO : Trimethylarsineoxide, TeMA: tetramethylated arsenic acid, GSH : Glutathione (reduced form), concentration of arsenic of the stating arsenic compound: 2.7nmol, GSH (Reduced type of Glutathione): 130 μmol, Methylcobalamin: 7.4 μmol, solvent (tris HCL buffer solution, pH 8) : 50 μL, light energy: 36J (5mW/cm$^2$, 2 hours), respectively. Furthermore, a conversion ratio was calculated by the following formula: A conversion ration (%) = 100% × (The concentration of arsenic after the reaction / The concentration of arsenic before the reaction).

A methylating reaction is shown in the table 10 as a reference example in the case of the use of a harmless TMAO as a stating material. As it is clear from the test numbers 82, 84, 86, 88, 90, 92, 94, 96, 98 and 100, it was revealed that the harmless TMAO maintains its stability in the present reaction conditions with no degradation into iAs (III), iAs (V), MMA or DMA by the exposure to light or the heating.

Industrial applicability

The present inventions make a significant contribution in the broad fields of treatments of the industrial waste etc., and environmental protections concerning a polluted mud or a soil, since the harmless compounds obtained by converting the harmful compound containing arsenic etc., to more harmless compound according to the method of the present invention, are extremely stable and safe.

## Claims

1. A method of detoxifying a harmful compound, wherein a harmful compound containing at least one element selected from the group comprising arsenic, antimony and selenium is detoxified by an exposure to light and / or a heating under the presence of a cobalt complex.

2. A method of detoxifying a harmful compound according to claim 1, wherein the harmful compound is detoxified by an alkylation of arsenic, antimony and selenium.

3. A method of detoxifying a harmful compound according to claims 1 or 2, wherein an alkylation reaction is carried out under an exposure to light and / or a heating.

4. A method of detoxifying a harmful compound according to any one of claims 1 to 3, wherein the harmful compound is detoxified under the presence of a reducing agent capable of reducing at least one metal selected from the group comprising arsenic, antimony and selenium.

5. A method of detoxifying a harmful compound according to claim 4, wherein the reducing agent is a material having SH group.

6. A method of detoxifying a harmful compound according to claim 5, wherein the material having SH group is at least one selected from the group comprising glutathione, reduced glutathione (GSH), cysteine, S-adenosyl cysteine, sulforaphane, homocysteine and thioglycol.

7. A method of detoxifying a harmful compound according to any one of claims 1 to 6, wherein the cobalt complex is methyl complex comprising at least one compound selected from methylcobalamin (methylated vitamin B12, official name: Coα-[α-5,6- dimethylbenz-1H-imidazole-1-yl-Coβ- methylcobamide]), vitamin B12 such as cyanocobalamin, cobalt(II) acetyl acetonate, cobalt(III) acetyl acetonate, cobalt carbonyl (dicobalt octacarbonyl), cobalt(II)1,1,1,5,5,5-hexafluoro acetyl acetonate, cobalt (II) meso-tetra phenyl porphin, hexafluoro phosphoric acid bis (pentamethyl cyclopenta dienyl) cobalt, N, N'-bis (salicylidene) ethylene diamine cobalt(II), bis (2,2,6,6-tetramethyl-3,5-heptanedionato) cobalt(II), (chlorophthalocyaninnato) cobalt(II), chlorotris (triphenylphosphine) cobalt(I), methyl complex of cobalt(II) acetate, cobalt(II) benzoate, cobalt(II) cyanide, cyclohexane cobalt(II) butyrate, 2-cobalt(II) ethylhexanoate, meso-tetramethoxyphenyl porphyrin cobalt(II), cobalt naphthenate, cobalt(II) phthalocyanine, methyl cobalt(III) protoporphyrin IX, cobalt stearate, cobalt(II) sulfamate, (1R, 2R)-(-)-1,2-cyclohexanediamino-N,N'-bis (3,5-di-t-butylsalicylidene) cobalt(II), (1S, 2S)-(+)-1,2-cyclohexanediamino-N,N'-bis (3, 5-di-t-butylsalicylidene) cobalt(II), cyclopentadienyl bis (triphenylphosphine) cobalt(I), cyclopentadienyl cobalt dicarbonyl, dibromo bis L(triphenylphosphine) cobalt(II), (tetraaminochloro phthalocyaninnato) cobalt(II), (tetra-t-butyl phthalocyaninnato) cobalt(II), or at least one selected from the group comprising cobalt-methyl complex formed by allowing the cobalt compound to coexist with the alkyl halide, especially methyl halide.

**8.** A method of detoxifying the harmful compound according to any one of claims 2 to 7, wherein the alkylation is a methylation.

**9.** A method of detoxifying the harmful compound according to claim 8, wherein the harmful compound is converted to a dimethyl compound, or trimethyl compound by the methylation.

**10.** A method of detoxifying the harmful compound according to claim 9, wherein the dimethyl compound is dimethyl arsonyl ethanol (DMAE), dimethyl arsonyl acetate (DMAA), dimethylarsinic acid, or arseno sugar.

**11.** A method of detoxifying the harmful compound according to claim 9, wherein the trimethyl compound is arsenocholine, arsenobetaine, trimethyl arseno sugar or trimethyl arsine oxide.

**INTERNATIONAL SEARCH REPORT**

International application No. PCT/JP2008/002566

A. CLASSIFICATION OF SUBJECT MATTER
*A62D3/30*(2007.01)i, *A62D3/176*(2007.01)i, *A62D3/37*(2007.01)i, *B09B3/00* (2006.01)i, *C02F1/70*(2006.01)i, *C02F11/00*(2006.01)i, *C07F15/06*(2006.01)i, *C07H23/00*(2006.01)i, *A62D101/43*(2007.01)n

According to International Patent Classification (IPC) or to both national classification and IPC

B. FIELDS SEARCHED

Minimum documentation searched (classification system followed by classification symbols)
A62D3/30, A62D3/176, A62D3/37, B09B3/00, C02F1/70, C02F11/00, C07F15/06, C07H23/00, A62D101/43

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
WPI, JSTPlus(JDreamII), JMEDPlus(JDreamII), JST7580(JDreamII), JAPICDOC(JDreamII), JCHEM(JDreamII)

C. DOCUMENTS CONSIDERED TO BE RELEVANT

| Category* | Citation of document, with indication, where appropriate, of the relevant passages | Relevant to claim No. |
|---|---|---|
| P,X | WO 2008/012953 A1 (Nippon Sheet Glass Co., Ltd.), 31 January, 2008 (31.01.08), Claim 36; examples 15 to 18 (Family: none) | 1-11 |
| P,X | WO 2008/012948 A1 (Nippon Sheet Glass Co., Ltd.), 31 January, 2008 (31.01.08), Claims 18 to 28; Par. Nos. [0098] to [0106] (Family: none) | 1-11 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 October, 2008 (23.10.08) | 04 November, 2008 (04.11.08) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No. PCT/JP2008/002566

C (Continuation). DOCUMENTS CONSIDERED TO BE RELEVANT

| Category* | Citation of document, with indication, where appropriate, of the relevant passages | Relevant to claim No. |
|---|---|---|
| A | WO 2007/088680 A1 (Nippon Sheet Glass Co., Ltd.), 09 August, 2007 (09.08.07), Claims 13 to 20; examples 1 to 4, 11 to 15 (Family: none) | 1-11 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Kaise et al.** *Organomet. Chem.,* 1998, vol. 12, 137-143